# EUROPEAN PATENT APPLICATION

(11) **EP 2 732 759 A1**
(43) Date of publication of application: **21.05.2014**
(21) Application number: 13192338.5
(22) Date of filing: 11.11.2013
(51) Int. Cl.: A61B 5/021, A61B 5/022, A61B 5/00, A61B 5/026, A61B 5/024

(54) **System and method of measurement of systolic blood pressure**

(30) Priority: 19.11.2012 IL 22312512
(71) Applicant: Jerusalem College of Technology, 93721 Jerusalem (IL)
(72) Inventor: Nitzan, Meir, 90631 Beth-El (IL)
(74) Representative: Jaeger, Michael David

(57) **Abstract**

Systolic blood pressure of a subject is determined by application of monotonic changing pressure conditions over a region of an organ of the subject, simultaneous illumination of a tissue in the pressurized organ with light and measurement of optical data indicative of passage of the light through the tissue and of pressure data indicative of the pressure being applied over said region of said organ. At least one pulsatile and at least one baseline component are determined from the measured optical data and changes are then identified in each of the components, said changes indicative that the pressure applied over the organ is smaller than a systolic blood pressure of the subject. The systolic blood pressure of the subject may be determined as a maximal applied pressure at which at least one of the changes in the pulsatile component and the changes in the baseline component started to appear.

## Description

### TECHNOLOGICAL FIELD

The present invention relates to evaluation of blood pressure of a subject by applying pressure on an artery in a limb of the subject and measuring the light absorption in a downstream tissue.

### BACKGROUND

The assessment of arterial blood pressure has both physiological and clinical significance, and tremendous efforts have been made to develop reliable noninvasive methods for measurement thereof. One common method for measuring blood pressure is manual sphygmomanometry, which is considered to be the most accurate noninvasive method, and to which other methods are usually compared. In manual sphygmomanometry an external inflatable cuff is utilized to apply a pressure greater than the systolic blood pressure over a limb followed by audible detection of Korotkoff sounds by a stethoscope while the cuff pressure over the limb is gradually released.

However, manual sphygmomanometry is also prone to several sources of errors, such as, for example, insufficient hearing acuity of the user and behavioral factors, which influence the level of blood pressure, such as the presence of a physician (also known as white coat hypertension). Some of these errors may be avoided when automatic measurement of blood pressure is performed, and several methods have been suggested for automatic noninvasive blood pressure (NIBP) measurement. The most widely used of these automatic NIBP methods are oscillometry and automatic auscultatory methods.

Automatic oscillometry, like manual sphygmomanometry, utilizes an external inflatable cuff and is based on the measurement of air pressure oscillations induced in the pressure cuff during cuff deflation due to cardiac activity. In automatic oscillometry, systolic blood pressure (SBP) and diastolic blood pressure (DBP) values are determined from the envelope of the oscillometric curve using empirical criteria. These empirical criteria are the main source of error in oscillometry since the envelope of the oscillometric curve does not depend merely on the SBP and the DBP, but also on the characteristics of the arteries under the cuff and also on characteristics of the cuff itself. In addition, the oscillometry pulses also appear for cuff pressure above the SBP value, even though the arteries under the pressure cuff are closed, due to the impact of the arterial blood on the tissue under the proximal (upstream) end of the cuff.

Auscultation methods are based on identifying the commencement of the Korotkoff sounds in the cuffed limb when the cuff pressure decreases below the SBP value and the arteries under the pressure cuff reopen. Automatic auscultatory methods detect the Korotkoff sounds using an electronic sound transducer and present blood pressure readings on a digital display. However, the automatic auscultation method is prone to artifacts mainly due to external noise and vibrations.

Accuracy of the available automatic NIBP meters used at present is low, as can be deduced from the standards imposed by the Association for the Advancement of Medical Instrumentation (AAMI) and the British Hypertension Society (BHS) (1-3). Both standards are based on comparing blood pressure values obtained in simultaneous measurements by the automated NIBP meter and manual sphygmomanometry (the reference standard) on subjects having wide ranges of blood pressure. According to both standards a device for which 5% of the examinations differ from the reference device by 15 mmHg or more is acceptable. Such low accuracy is permitted because the known methods are not capable of providing automatic blood pressure measurements of higher accuracy.

In small babies, and in particular neonates, Korotkoff sounds are faint, or altogether absent, and manual sphygmomanometry is not always suitable for accurate assessment of blood pressure. In neonates, the common method for the measurement of blood pressure is oscillometry, despite its low accuracy.

Several techniques for the measurement of SBP are based on the detection of a physiological effect which starts to appear during the cuff deflation period, only when the cuff pressure decreases below the SBP value, like the Korotkoff sounds. These physiological effects include manual palpation of a superficial artery, laser Doppler flowmetry, Doppler ultrasound or photoplethysmography (PPG).

The PPG-based SBP measurement methods utilize a pressure cuff wrapped around the arm of the examined subject and a distal PPG sensor optically coupled to a finger, downstream to the cuff, for measuring PPG signals therein. The measured PPG signals are indicative of changes in light transmission through the tissue of the subject due to the increase and decrease of arterial blood volume during the systolic (heart contraction) and diastolic (heart relaxation and dilatation) periods respectively, in the arteries downstream to the location of the cuff.

In the PPG-based method the cuff pressure is raised to above the SBP, and thereafter the cuff pressure is slowly released. For cuff pressure above the SBP, the artery under the cuff location is closed during the whole cardiac cycle, and therefore the distal PPG pulses disappear. At cuff pressures below the SBP, blood can pass through the artery under the cuff during that part of the cardiac cycle in which the arterial blood pressure is higher than the cuff pressure. Thus, the systolic blood pressure may be determined from the value of the cuff pressure at which the PPG pulses reappeared in the arteries downstream to the cuff. The problem with the PPG-based methods is that at cuff pressures slightly below the SBP, the measured PPG pulses are of small amplitude *(i.e.,* weak), since blood can pass through the artery under the cuff only during a small part of the cardiac cycle.

SBP is determined from the air pressure in the cuff at which the PPG pulses reappeared during cuff deflation. However, it has been found that when the cuff is inflated at high rate, 10-20% of subjects show no pulses in the light transmission curve until the pressure has decreased significantly beyond the actual SBP (as measured by Korotkoff sounds). This effect originates from the collapse of the finger arteries under the PPG sensor due to their drainage into the veins when the cuff air pressure is above SBP. In some cases the small blood volume pulses entering the arteries distal to the cuff when the cuff air pressure is slightly below the SBP value cannot open the collapsed arteries under the sensor. A possible solution to this phenomena is described in US patent No. 6,402,696, that was co-invented by the inventor of the present application, that proposes to reduce the drainage of blood from the arteries by increasing cuff pressure during the cuff inflation to above the SBP value at a relatively slow rate, in order to avoid drainage of the blood from the arterial circulation and prevent possible collapse of the small arteries.

US patent No. 7,544,168, that was co-invented by the inventor of the present application, discloses a cuff-based method for improving the detection of the PPG pulses at cuff pressures slightly below the SBP, by using two PPG sensors, one of which is located distal to the pressure cuff and the other not distal to it. The former was aimed to detect the reappearance of the PPG signals when the cuff pressure decreases below SBP value, and the role of the latter was to define time segments during which a PPG pulse would be expected to occur in the output of the distal-to-the-cuff detector, accounting for the time delay in the PPG signal distal to the pressure cuff (as explained by Nitzan et al., "Effects of external pressure on arteries distal to the cuff during sphygmomanometry" IEEE Tr. BME. 52:1120-1127, 2005). The technique also included a decision algorithm for automatic determination whether the signal which appeared in the output of the distal-to-the-cuff detector during the time segments defined by the second sensor, is in fact a PPG signal or is a noise in the light transmission curve. The decision algorithm is also described in a paper by Nitzan et al. "Automatic noninvasive measurement of systolic blood pressure using photophlethysmography". BioMedical Engineering OnLine, 8:28, 2009.

The PPG signal in the fingers, toes, hands and feet generally has high signal-to-noise ratio when measured in a limb free of pressure cuff, but has often only small amplitude in the PPG pulses appearing at cuff pressures slightly below the SBP value, (when the artery is open for only a short time during the cardiac cycle). The small amplitude of the first PPG pulses makes it difficult to reliably differentiate the reappearance of the PPG pulses from the background noise in the light transmission curve.

There is therefore a need for techniques for measuring arterial SBP using a cuff and a PPG sensor with improved accuracy, and for PPG sensor configurations capable of providing higher signal-to-noise ratio than that of the regular PPG sensors, and in particular higher amplitude of the first PPG pulses reappearing after the cuff air pressure decreases below the SBP value.

### GENERAL DESCRIPTION

The invention generally relates to techniques for determining systolic blood pressure in a subject by applying descending pressure conditions (e.g., by a pressure cuff) over part of an organ of the subject (e.g., an arm or other limb, also referred to herein as pressurized region or organ) starting from a pressure level greater than the systolic blood pressure and simultaneously illuminating a tissue downstream to the pressurized region with light, measuring optical data indicative of passage of the light through the tissue and pressure data indicative of the pressure being applied over the organ, analyzing the measured optical data to determine at least one pulsatile component and at least one slowly changing component (hereinafter DC or baseline component) thereof, identifying in each of the components changes indicative that the pressure applied over the organ is smaller than a systolic blood pressure of the subject (*i.e*., indicative of increase of blood volume in the tissue downstream the pressurized region), and determining for each pulsatile and DC component the value of the applied (air or cuff) pressure at which the changes associated with the component started to appear. e.g., a maximal applied (air or cuff) pressure at which the changes associated with the specific component reappeared.

In some embodiments the systolic blood pressure of the subject is determined based on the maximal pressure values determined for each pulsatile and DC component. For example, in some embodiments the systolic blood pressure of the subject is determined as the pressure applied over the organ at a time at which the changes in at least one of the pulsatile and DC components started to appear. In other words, in some embodiments, the systolic blood pressure of the subject is determined to be the air pressure applied over the organ at which a change indicative that the applied pressure declined to a level smaller than the systolic blood pressure of the subject was identified for the first time by a change in one of the pulsatile and DC components.

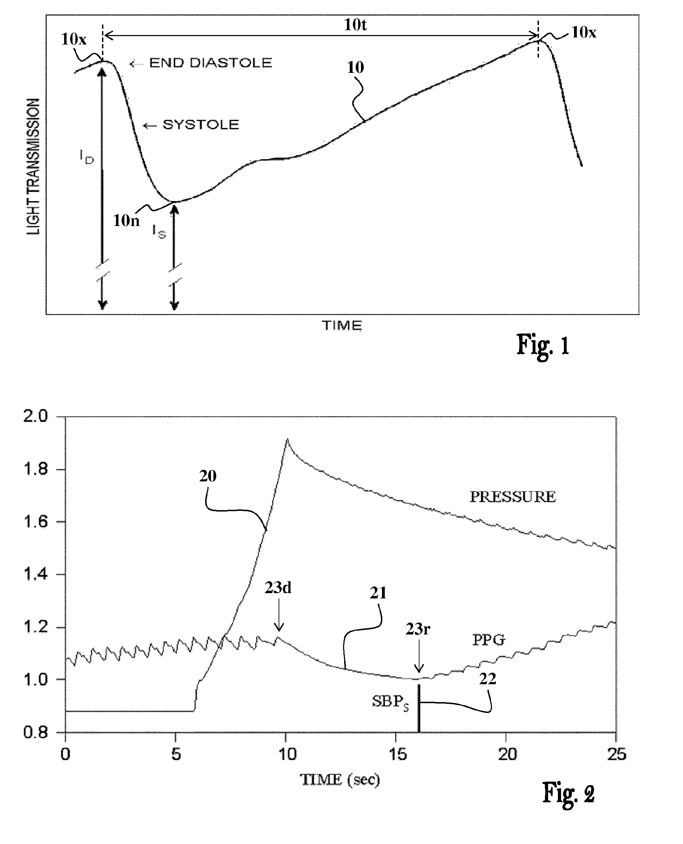

For better understanding of the invention, a brief description of some principles and terms is provided in the sections below. Fig. 1 shows a PPG signal 10 measured in a finger (also referred to herein as *examined tissue*) of a subject's limb free of pressure cuff *(i.e.,* no pressure is applied over the limb). As seen in **Fig. 1** the light transmission through the examined tissue varies over time, which reflects blood volume variations in the arteries in the examined tissue. In the PPG-signal **10** the maximal intensity of transmitted light, *I_{D}*, occurs at the end of the diastolic phase (**10x**, when the tissue blood volume is at minimum), and the intensity of transmitted light decreases during the systolic phase (when blood volume increases) and reaches a minimum, *I_{S}*, occurring at the end of the systolic phase (**10n**). Accordingly, a PPG pulse may be defined as the PPG signal persisting in the time region **10t** between consecutive maximums **10x.** The DC value (also referred to herein as baseline value) for each pulse may be defined as: (i) the minimum measured light intensity ***I_{S}*** of the PPG pulse; (ii) the maximum measured light intensity of the PPG pulse ***I_{D}*;** or (iii) an average value of measured light intensities of the PPG pulse, during the PPG pulse period. The DC component (also referred to herein as baseline component or trend) of the PPG signal is defined as the light transmission curve after the application of a low-pass filter (such as an electronic RC circuit or digital moving average filter) that filters out the PPG pulses. The AC component (also referred to herein as pulsatile component) of the PPG signal is the PPG signal after subtracting the DC component from it. The amplitude of the PPG pulse may be generally defined by the subtraction of the maximal (diastolic) intensity from the minimal (systolic) intensity *e.g., **I_{D}-I_{S}.***

**Fig. 2** shows a PPG signal **21** in a finger distal/downstream to a pressure cuff wrapped around an arm of an examined subject and a curve **20** of the pressure in the cuff. For cuff pressure above the SBP value the PPG signal disappears (at **23d),** and reappears when the cuff pressure decreases below the SBP value (at **23r).** It should be noted, however, that at cuff pressures slightly below the SBP, the artery under the cuff is closed in most of the cardiac cycle, and is open only when the arterial blood pressure increases above the cuff pressure, so that only small amount of blood can pass through the arteries downstream to the cuff in each heart beat, thereby generating small pulses in the light transmission curve. The time at which the cuff pressure decreased to the SBP value, as determined by sphygmomanometry (SBP_{S}) is marked by a bold line identified by numeral reference **22.**

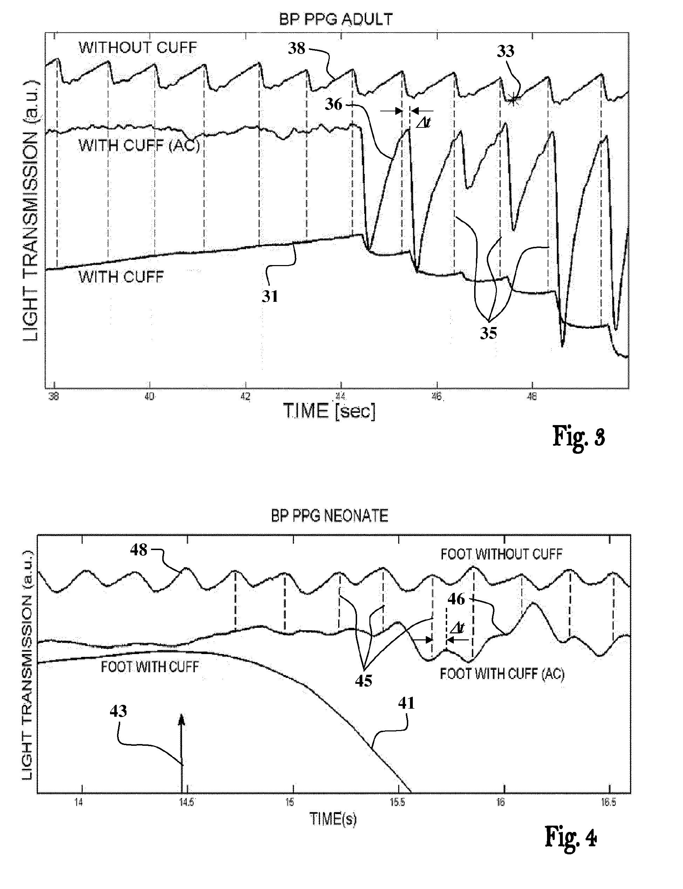

**Fig. 3** and **Fig. 4** present the raw light transmission curves for an adult **(31,** in **Fig. 3****)** and for a neonate **(41,** in **Fig. 4****),** respectively, during the decrease of cuff pressure, measured by a PPG probe (*e.g*., comprising a light source and light detector optically coupled to the examined tissue) downstream to the cuff after raising the cuff pressure to a pressure above the SBP value and gradually releasing the cuff pressure to a pressure below the SBP. In both examples the light transmission gradually decreases after the cuff pressure decreases below the SBP value. The time at which the cuff pressure decreased to the value of the SBP, as obtained by auscultatory sphygmomanometry is identified in **Fig. 3** by asterisk **33,** and in **Fig. 4** by arrow **43** as obtained by oscillometry. The decrease of light transmission is due to the gradual increase of blood volume in the pressurized organ, due to entrance of arterial blood during systole without draining by the veins, which are closed by the cuff.

The DC component (trend) of the light transmission curve is obtained by smoothing the light transmission curve over several pulses, so that the PPG pulses are eliminated. After subtraction of the trend of the light transmission curves from the light transmission curves themselves, and magnifying the scales, it is possible to see more clearly the PPG pulses, when the cuff air pressure decreased below SBP value. These PPG pulses can be seen in the AC curve **36** shown in **Fig. 3** for an adult, and in the AC curve **46** shown in **Fig. 4** for a neonate. Both the decrease in the light transmission curves and the reappearance of the PPG pulses in the AC curves when the cuff pressure decreases below the SBP value can be used for the determination of the SBP value for the examined subject/neonate. However, the PPG pulses measured at cuff pressures slightly below the SBP are small and faint, and in many cases it is difficult to detect them on the background of noise in the AC curve. For the same reason the decrease in the light transmission curves at cuff pressures slightly below the SBP is small, and in many cases it is difficult to detect it on the background of low frequency noise in the light transmission curve.

**Fig. 3** and **Fig. 4** also show the PPG curves obtained by a second PPG probe attached to cuff-free organ of the examined subject/neonate. More particularly, curve **38** in **Fig. 3** shows cuff-free PPG signals obtained from a finger in the other hand of the (adult) subject, and curve **48** in **Fig. 4** shows cuff-free PPG signals obtained from the other foot of the neonate. These cuff free PPG signals are used in some embodiments as a time reference to more accurately identify time regions in which the PPG pulses distal (downstream) to the cuff were expected to reappear.

The vertical dashed lines **(35** and **45** in **Fig. 3** and **Fig. 4****,** respectively) show the start of the decrease in the systolic pressure in the PPG pulses measured in the limb free of cuff pressure. As seen, the PPG pulses distal to the cuff appear with time delay ***Δt*** relative to those in the cuff-free organ. This delay ***Δt*** results from changes in the hemodynamic properties of the arteries under the cuff or distal to the cuff as a result of the inflation and deflation of the cuff, and has to be considered when determining the time regions in which the PPG pulses distal (downstream) to the cuff are expected to reappear.

The amplitude of the first reappearing PPG pulses for cuff pressure slightly below the SBP is small, often making it difficult to identify them reliably from the noise in the light transmission curve. To overcome this difficulty several proposed techniques suggested using the decrease of DC light transmission curve after the air pressure falls below the SBP value for the detection of the SBP. For example, for patients of low blood pressure, US Patents Nos. 5,485,838 and 5,676,140, and also US Patent No. 5,447,161 which suggests measurement of the systolic blood pressure from the change in the slope of the PPG DC curve during the increase of the cuff pressure.

The detection of the decrease in the DC light transmission curve is preferable to the detection of the PPG pulses in cases of noise in the frequency range of the cardiac cycle, *i.e.,* 0.5-3 Hz (the existing frequency range of the PPG pulses), but has lower reliability in cases wherein the light transmission curve has noise fluctuations of lower frequency. Similar to the reappearance of the PPG pulses, at cuff pressures slightly below the SBP the magnitude of the rate of decrease in the DC light transmission curve is small, since blood can pass through the arteries under the cuff only during a small part of the cardiac cycle.

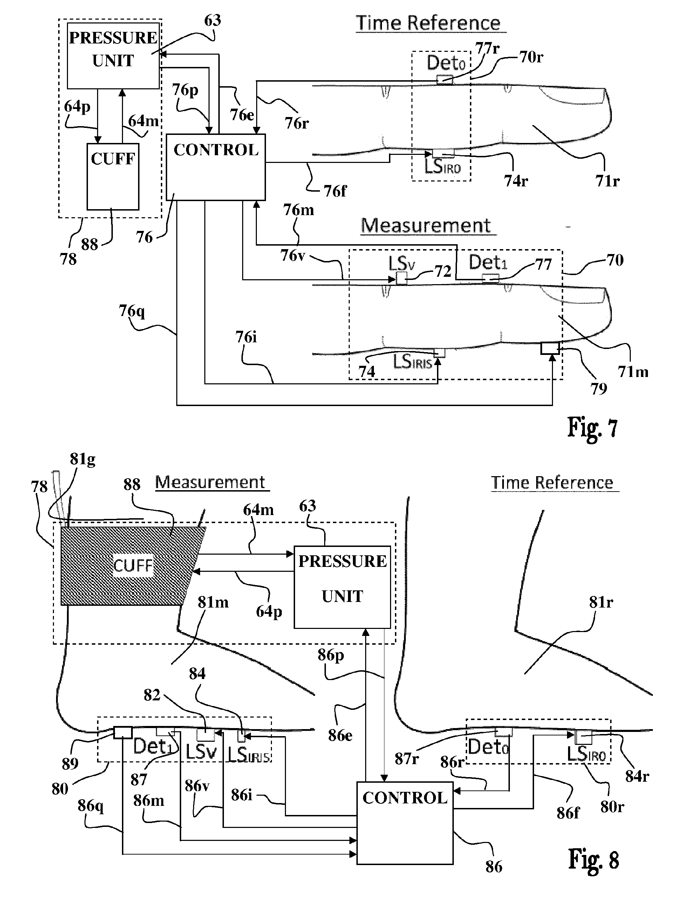

In possible embodiments the PPG probe comprises a single light detector (e.g. PIN diode) and a single light source (hereafter also referred to as a first light source e.g., LED) configured to illuminate the examined tissue with light wavelength near an infrared isosbestic point (*e.g.,* in the range of 790 to 815 nanometer wavelength range, optionally about 800 nanometers). The light source may be configured to operate in a transmission-mode (*e.g*., finger probe **70** as exemplified in **Fig. 7**), or placed at a distance of about 10 to 20 millimeters from the detector in a reflection-mode (*e.g.,* foot probe **80** as exemplified in **Fig. 8****).** This light source and light detector pair PPG probe configuration may be operated by a control unit in conjunction with a pressure device, as described herein above and below, wherein the control unit is configured to receive optical data indicative of passage of the illuminated light through the examined tissue as measured by the light detector, and pressure data indicative of the pressure applied over the organ by the pressure device, analyze the optical data to determine pulsatile and baseline components thereof, and identify the first appearance of a change indicative of increase of blood volume in the pressurized organ in the pulsatile and the baseline components. The systolic blood pressure of the examined subject may be determined by the control unit as the pressure at which the change indicative of increase of blood volume in the pressurized organ appeared in one of the determined components for the first time.

In some embodiments the PPG probe may further include a second light source configured to operate in the visible wavelength range (*e.g.,* 400 to 600 nanometer wavelength range, preferably in the range of 450 to 500 nanometer wavelength range). The second light source may be located a few millimeters from the light detector (*e.g.,* about 2 to 7 millimeters) and configured to operate in a reflection-mode for detecting the reappearance of the PPG pulses when the cuff pressure decreases below the SBP value. Accordingly, the optical data obtained via the light detector in this example is indicative of light in the wavelength ranges of the first and second light sources passing through the examined deep and superficial tissues, respectively. The optical data may be analyzed by the control unit to determine pulsatile and baseline components either for each of the illumination of light wavelengths or for their integrated effect.

Optionally, the control unit may be configured to determine only pulsatile and baseline components derived from the reflected/transmitted light illuminated by the second light source. The determined pulsatile and baseline components may be then analyzed to determine from the pressure data for each specific pulsatile and baseline component a maximal measured pressure at which changes indicative of increase of blood volume downstream the pressurized region started to appear for the first time in the specific component. The systolic blood pressure of the subject may be determined based on the maximal pressures determined for each specific pulsatile and baseline component. For example, in some embodiments the systolic blood pressure of the subject is determined as the highest pressure applied over the organ at a time at which the changes in at least one of the pulsatile and baseline components started to appear.

In some embodiments the PPG probe may comprise a third light source configured to operate in the red spectral region (e.g., in the 620 to 740 nanometer wavelength range), and which may be combined with the first light source to form a single light-source capable of illuminating light in two spectral ranges. In this example the control unit may be configured to determine pulsatile and baseline components to some, or all, of the illumination of light wavelengths, and discriminate changes identified in the pulsatile components determined for the light in the near isosbestic wavelength point which are due to background noise from changes that are due to increase of blood volume in the pressurized organ. Similarly, the systolic blood pressure may be determined based on the maximal pressures determined for each one of the pulsatile and baseline components e.g., the highest pressure at which the change indicative of increase of blood volume downstream the pressurized region has been identified in one of the determined components for the first time.

In some possible embodiments the control unit is configured to intermittently operate the light sources by employing a time sharing scheme (e.g., multiplexing), receive from the light detector respective signals indicative of measured light transmission/reflectance through/from the examined tissue, and generate a respective PPG curve for each of the light wavelengths illuminations. Alternatively, one or more additional light detectors may be used to collect the transmitted/reflected light. For example, in a possible embodiment the PPG probe may include a plurality of light detectors, each configured to measure light transmission/reflectance of a specific one of the light sources of the PPG probe.

In possible embodiments the control unit may be configured to operate the PPG probe and generate the PPG curves for the different light wavelengths by employing a frequency modulation operation scheme.

In possible embodiments identifying a change in pulsatile and baseline components (*e.g.,* reappearance of the PPG pulses or commencement of declination of the DC signal) is carried out using one or more suitable algorithms (*e.g.,* as described in US patent 7,544,168 and/or by Nitzan et al, in "Automatic noninvasive measurement of systolic blood pressure using photophlethysmography", BioMedical Engineering OnLine, 8:28, 2009), configured to detect pulses or curve declination in a slowly changing signal.

In some embodiments the detection of the changes in the pulsatile and baseline components is carried out visually by offline inspection of the light transmission curves, *(e.g.,* for the detection of the PPG pulses in the pulsatile component or the commencement of declination in the baseline component).

In some embodiments two different techniques for determining systolic blood pressure are performed simultaneously, employing light transmission/reflectance measurements and sphygmomanometry, which is based on auscultation of Korotkoff sounds. The identifying of a change in one of the pulsatile and baseline components during the gradual decrease of the pressure applied over the body organ may be carried out visually by offline inspection of the light transmission curves. The control unit may be configured to determine the systolic blood pressure as the highest pressure applied over the organ at a time at which the changes in at least one of the Korotkoff sounds, the PPG pulsatile and baseline components, started to appear. In other words, the control unit may be configured to determine the systolic blood pressure as the highest pressure applied by the pressure device at which the detection of Korotkoff sounds or the detection of the reappearance of the PPG pulses or the detection of the commencement of declination in the light transmission/reflectance was determined.

In some embodiments two techniques for determining systolic blood pressure are utilized simultaneously including measurement of light transmission/reflectance through/from the examined tissue and sphygmomanometry, which is based on automatic detection of Korotkoff sounds. The identifying of a change in one of the pulsatile and baseline components during the gradual decrease of the pressure applied over the body organ may be carried out automatically offline by analyzing the light transmission/reflectance curves. The control unit may be configured to determine the systolic blood pressure as the highest pressure applied by the pressure device at which the detection of Korotkoff sounds or the detection of the reappearance of the PPG pulses or the detection of the decrease in the light transmission was determined.

In some embodiments the rate of change of the applied descending pressure conditions may be in the range of 1 to 5 mmHg/sec, preferably about 1 to 3 mmHg/sec, in order to increase the accuracy of the pressure measurement.

In some embodiments the pressure applied by the pressure device is raised to above the SBP value in relative slow rate, in the range of 10 to 15 mmHg/sec, in order to avoid drainage of the blood from the arterial circulation and possible collapse of the small arteries.

In some embodiments systolic blood pressure is measured during ascending pressure period. The control unit may be configured to determine the systolic blood pressure as the highest pressure applied by the pressure device at which the detection of Korotkoff sounds or the detection of the reappearance of the PPG pulses or the detection of the decrease in the light transmission was determined. The rate of change of the applied descending pressure condition may be in the range of 1 to 5 mmHg/sec, preferably about 1 to 3 mmHg/sec, in order to increase the accuracy of the pressure measurement.

In some embodiments another PPG probe is utilized to simultaneously obtain PPG signals from a tissue site in which the blood circulation is not stopped by the cuff pressure (e.g., in a contralateral limb or upstream the cuff) used as a time-reference for the differentiation of the reappearance of the PPG pulses from the background noise. Alternatively, or additionally, pressure pulses in the cuff or the ECG R-wave may also be used as a time-reference.

According to one aspect of the present application there is provided a system for determining systolic blood pressure of a subject, the system comprising a pressure device configured and operable to apply monotonic (*i.e.*, having a fixed trend) changing pressure conditions (*i. e.* either ascending or descending) over a region of an organ of the subject, and generate pressure data indicative of the pressure applied over the region of the organ, an optical probe comprising at least one light source configured and operable to illuminate light over a downstream (*i.e.*, distal to the pressure device) tissue in the organ with light, and at least one light detector configured and operable to detect light response of the tissue to the illuminated light and generate optical data indicative thereof, a control unit configured and operable to operate said pressure device to apply the pressure conditions over a range of pressures including the systolic blood pressure over the region of the organ and simultaneously operate the optical probe to illuminate the tissue and generate the responsive optical data, to analyze the optical data and determine at least one pulsatile component and at least one baseline component therefrom, identify in said pulsatile and baseline components appearance of changes related to heart-induced change in (*e.g.,* increase of) blood volume in the organ in each one of the pulsatile and baseline components, and determine the systolic blood pressure of the subject from the pressure data as a highest pressure value at which at least one of the identified changes started to appear.

For example, the control unit may be configured and operable to determine from the pressure data a highest pressure value for each of the pulsatile and baseline components at which the identified changes are related to the blood volume changes in the organ, and determine the systolic blood pressure as the maximum value from the determined highest pressure values.

In some embodiments descending pressure conditions are applied over the region of the organ starting from a pressure greater than the systolic blood pressure. Accordingly, when the descending pressure conditions are being applied, the control unit is configured and operable to determine from the pressure data a highest pressure value for each of the pulsatile and baseline components at which the identified changes are responsive to increase of blood volume in the organ.

In some embodiments, ascending pressure conditions are applied over the region of the organ starting from a pressure smaller than the systolic blood pressure. Accordingly, when the ascending pressure conditions are being applied, the control unit is configured and operable to determine from the pressure data a highest pressure value for each of the pulsatile and baseline components which are related to change of blood volume in the organ.

The optical probe may comprise a light source configured and operable to illuminate light in a wavelength near an infrared isosbestic wavelength point, and/or a light source configured and operable to illuminate light in the visible wavelength range (e.g., in the 450-506 nm wavelength range). The visible wavelength light source may be situated at a distance of about 2 to 7 millimeters from the light detector and configured and operable to operate in a reflection-mode. The near isosbestic wavelength point light source may be configured and operable to operate in a transmission mode.

In some embodiments the control unit is configured and operable to determine only the at least one pulsatile component responsive to the visible light illumination.

The system may include an additional optical probe comprising at least one light source configured and operable to illuminate a tissue (*e.g.,* in a wavelength near or above an isosbestic wavelength point) in another organ, or another organ site, of the subject, not being affected by the pressure applied by the pressure device, and at least one light detector configured and operable to detect light response of said tissue to the illuminated light and generate reference optical data indicative thereof, wherein the control unit is configured and operable to determine a pulsatile component from the reference optical data and utilize it to discriminate between pulsatile changes which are responsive to the heart-induced change in blood volume in the organ and changes that are due to noise.

In some possible embodiments the optical probe comprises at least one light source configured and operable to illuminate light in a wavelength above the infrared isosbestic wavelength point (*e.g.,* in the 850-900 nm range). Optionally, the above isosbestic wavelength point light source is configured and operable to operate in a transmission-mode. The control unit may be configured and operable in some embodiments to determine the at least one pulsatile component responsive to the above isosbestic wavelength point illumination of infrared light and the at least one baseline component responsive to the near isosbestic wavelength point illumination of infrared light.

In some applications the system includes a sound transducer configured and operable to sense audible signals in the organ region while the changing pressure conditions are being applied and generate audible data indicative thereof, wherein the control unit is configured to process the audible data and detect Korotkoff sounds therein. The control unit may be configured to determine from the pressure data the highest pressure at which the Korotkoff sounds started to appear or disappear and determine the systolic blood pressure as the maximum pressure value from the highest pressure values determined for the pulsatile component, the baseline component, and the Korotkoff sounds. For example, in some embodiments the control unit is configured and operable to determine the systolic blood pressure of the subject as a maximal applied pressure at which at least one of the following events occurred: appearance or disappearance of the Korotkoff sounds; the changes in the pulsatile component, and the changes in the baseline component.

In some possible embodiments all of the light sources are configured and operable to operate in a reflection-mode.

According to another aspect of the present application there is provided a method of determining systolic blood pressure in a subject, the method comprising applying monotonic changing (*e.g.,* ascending or descending) pressure conditions over a region of an organ of the subject over a range of pressures including the systolic blood pressure and simultaneously illuminating a tissue downstream the pressurized region with light, measuring optical data indicative of passage of the light through the tissue and pressure data indicative of the pressure being applied over the organ, determining at least one pulsatile component and at least one baseline component from the measured optical data, identifying in the pulsatile component and in the baseline component changes indicative that the pressure applied over the organ is smaller or greater than a systolic blood pressure of the subject, and determining the systolic blood pressure of the subject as a maximal applied pressure at which at least one of the following started to appear: the changes in the pulsatile component; and the changes in the baseline component.

The method may further comprise illuminating another tissue (not being under the applied pressure conditions) of the subject with light, measuring optical data indicative of passage of the light through said another tissue, determining an additional pulsatile component from the optical data measured from said another tissue and using it as a reference to discriminate the changes indicative that the pressure applied over the organ is smaller or greater than the systolic blood pressure of the subject from noise in the pulsatile component measured for the tissue in the organ to which the changing pressure conditions are being applied.

Optionally, the method comprises determining a time delay between pulses in the pulsatile components measured for the tissue in the organ to which the changing pressure conditions are being applied and the pulsatile components measured for the tissue not being under the applied pressure conditions, and aiding the discrimination of the changes from the noise with said time delay.

In some possible embodiments the method comprises detecting Korotkoff sounds in the organ to which the changing pressure conditions are being applied, wherein the determining includes determining the systolic blood pressure of the subject as a maximal applied pressure at which at least one of the following started to occur: appearance or disappearance of said Korotkoff sounds; the changes in the pulsatile component, and the changes in the baseline component.

### BRIEF DESCRIPTION OF THE DRAWINGS

In order to understand the invention and to see how it may be carried out in practice, embodiments will now be described, by way of non-limiting example only, with reference to the accompanying drawings, in which like reference numerals are used to indicate corresponding parts, and in which:
**Fig. 1** shows a PPG signal;
**Fig. 2** shows a curve of a PPG signal taken from a finger to the corresponding arm on which varying pressure conditions are applied by a cuff, and a curve of the cuff pressure as a function of time to demonstrate the reappearance of the PPG signals after cuff pressure decreases to below the SBP value;
**Fig. 3** shows raw light transmission and AC PPG signal curves taken from a finger on one hand of an adult subject during the deflation of a cuff situated over the corresponding arm and a cuff free PPG signal simultaneously taken from a finger on the other hand of the subject;
**Fig. 4** shows raw light transmission and AC PPG signal curves taken from a foot of a neonate during the deflation of a cuff situated over the corresponding ankle and a cuff free PPG signal simultaneously taken from the other foot of the neonate;
**Fig. 5** shows extinction coefficient plots of the oxi- and deoxi-hemoglobin as a function of the wavelength, in the visible and near-infrared regions;
**Fig. 6** is a block diagram of a PPG-based blood pressure measurement device according to some possible embodiments;
**Fig. 7** schematically illustrates a PPG probe designed to measure PPG signals from a finger of a subject according to some possible embodiments;
**Fig. 8** schematically illustrates a PPG probe designed to measure PPG signals from a neonatal foot according to some possible embodiments; and
**Fig. 9** is a flowchart demonstrating a PPG-based blood pressure measurement process according to some possible embodiments.

It is noted that the embodiments exemplified in the figures are not intended to be in scale and are in diagram form to facilitate ease of understanding and description.

### DETAILED DESCRIPTION OF EMBODIMENTS

The present disclosure provides improved PPG-based techniques for measuring systolic arterial blood pressure in a subject. It is a principal object of the present invention, in some of its embodiments, to provide techniques to accurately detect the reappearance of the PPG pulses, and/or the start of declination of the light transmission curve, once pressure applied over the pressurized region of an organ is reduced below the SBP value. For this purpose, a new PPG probe was designed for measuring light transmission (and/or reflection) through (and/or from) the examined tissue.

The techniques disclosed herein utilize a system comprising a pressure applying device (also referred to herein as *pressure device)* comprising a pressure pump and a pressure applying element (also referred to herein as *pressure element)* configured to apply ascending and descending pressure conditions over a region of an organ (*e.g.,* an arm or other limb) of an examined subject, reaching a pressure level greater than the systolic blood pressure, and generate pressure data indicative thereof, a PPG probe configured to measure passage of light through an examined tissue (distal to the pressure element) and generate optical data indicative thereof, and a control unit configured to simultaneously operate the pressure device and the PPG probe, process the pressure data from the pressure element and the optical data from the PPG probe, and calculate one or more blood pressure indications (*e.g.,* SBP) based thereon.

The PPG probe is configured in some embodiments to optically couple to a tissue in the pressurized organ, distal (*i.e.*, downstream) to the pressure element, illuminate the examined tissue with light of one or more different wavelengths, measure the transmission and/or reflection of the light through/from the examined tissue for each of the different wavelengths, and generate optical data indicative thereof. The control unit is configured in some embodiments to activate the pressure device to increase the pressure applied over the organ to a pressure greater than the SBP and then to gradually release the pressure applied over the organ, simultaneously activate the PPG probe to illuminate the examined tissue with the light of one or more wavelengths and provide responsive optical data, and determine one or more blood pressure indications based on the pressure data and the optical data.

In some possible embodiments a combination of one or more of the following techniques are used to measure the blood pressure of a subject using the systems described hereinabove.

The following technique is designed to improve the PPG-based measurement and thus to increase the amplitude of the PPG pulses obtained from the tissue downstream the pressurized region of the organ at cuff pressures slightly below the SBP. For this purpose, in some possible embodiments, the PPG probe may comprise a light-source configured to illuminate light in wavelengths which are highly absorbed by oxygenated hemoglobin, which is the main constituent of hemoglobin in the arteries, and which is increased in the arterial blood volume during the systolic phase.

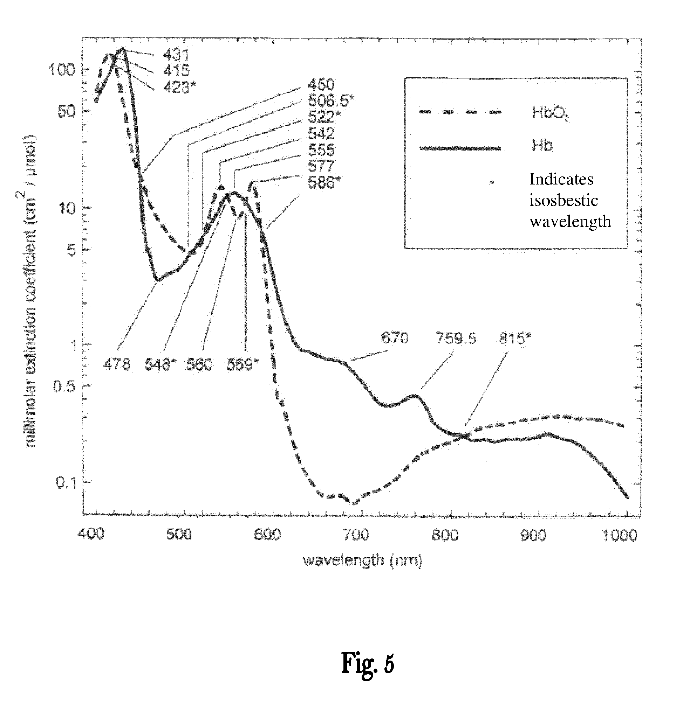

The extinction plots shown in **Fig. 5** illustrate the extinction coefficients (the ratio of absorption constant to the concentration in mol/cc) of oxygenated (HbO₂) and deoxygenated (Hb) hemoglobin as a function of the light wavelength. It can be seen that visible light in the spectral range of 400-600 nm is highly absorbed by hemoglobin. It is considered an advantage if the illuminating light has lower extinction coefficient for deoxygenated hemoglobin than for oxygenated hemoglobin in order to have higher transmission of light through the tissue. As seen in **Fig. 5** these two criteria are met in the spectral range of 450-506 nanometer wavelengths. Accordingly, in some embodiments, the PPG probe **(70** or **80** shown in **Fig. 7** and **Fig. 8** respectively) is configured to illuminate light in the visible range (e.g., 450-506 nm range) and measure the reflected light from the examined tissue.

Due to the high absorption of light in the 450-506 nanometer spectral range in hemoglobin, the distance between the light sources in the visible range (LS_{V} **72** and **82** in **Fig. 7** and **Fig. 8** respectively), and the respective detectors **(77** and **87),** should be relatively small, optionally about few millimeters *e.g.,* 2 to 7 mm, resulting in superficial light penetration region. The illumination of the examined tissue with low-depth penetration light in the visible range mainly provides indication for light passage through the skin, which in several clinical situations has low perfusion and low systolic increase in arterial blood volume. Therefore, in another technique as employed in some embodiments, another light source is used simultaneously to illuminate (in continuous or multiplex form) the examined tissue with light in the wavelength spectrum near 800 nanometers or above it (also referred to herein as IR light), which has relatively low extinction coefficient for both the oxygenated and the deoxygenated hemoglobin. The IR light source is applied either in reflection mode at a distance of about 20 mm from the light detector or in transmission mode, to allow transmission through high arterial blood volume, in order to increase the PPG signal.

Emission spectrum above the isosbestic point of 800 nanometers has higher extinction coefficient for oxygenated hemoglobin than for deoxygenated hemoglobin and thus may be used to obtain PPG pulses (AC) having higher amplitudes. Selection of emission spectrum in the neighborhood of the isosbestic point of 800 nanometers is advantageous for reducing noise in the DC curve, as will be explained hereinbelow.

As discussed hereinabove, the SBP value can also be obtained by identifying the start of declination of the baseline component of the raw light transmission curve. For cuff pressure above the SBP, the arteries and veins under the cuff are closed during the whole cardiac cycle, and blood cannot enter or drain from the tissue distal to the cuff. Though the blood volume is constant, the light transmission can change because the oxygenated hemoglobin can become deoxygenated during the time period in which the pressure conditions are being applied over the organ. The raw curves **31** and **41** shown in **Fig. 3** and **Fig. 4** respectively show the transmitted infrared light through a finger of an adult and a foot of a neonate distal to the cuff, during gradual decrease of the cuff pressure, after being raised to above systolic blood pressure. At cuff pressure above the SBP value, light transmission increases with time due to deoxygenation of hemoglobin, which lowers its extinction coefficient value relative to that for oxygenated hemoglobin, for light in the infrared region above the isosbestic point.

Hence, there are two effects that can change light transmission through the tissue under examination: change in blood volume, which is the required signal, and change in hemoglobin oxygenation, which is noise. The latter can be reduced by using light of the isosbestic wavelength, in which the values of the extinction coefficient for oxygenated hemoglobin and for deoxygenated hemoglobin are equal. However the LED light sources have relatively broad spectra and the value of the isosbestic wavelength is not exactly known, and according to different studies it has different values in the range of 798-815 nm (Kim and Liu, Phys. Med. Biol. 52: 6295-6322, 2007); hence in order to be practical, the light source is so chosen that its light spectrum has peak wavelength in the neighborhood of the isosbestic wavelength. An isosbestic wavelength suitable for this purpose may be in the neighborhood of 800 nanometers, because the slopes of the curves of the extinction coefficient as a function of wavelength are moderate both for oxygenated hemoglobin and for deoxygenated hemoglobin (as seen in Fig. 5). In this spectral region light transmission does not significantly change due to changes in the oxygen saturation of the blood in the tissue, and light transmission changes are mainly due to blood volume changes. Accordingly, in some possible embodiments the PPG probe is configured to measure the passage of light in the 800 nm range through the examined tissue for identifying DC curve declination with improved accuracy.

**Fig. 7** and **Fig. 8** demonstrate PPG probes, **70** and **80,** according to some possible embodiments, designed for measuring PPG signals in a finger **71m** of a subject, and in the foot **81m** of a neonate, respectively. Reference PPG probes, **70r** and **80r** respectively, may be used in possible embodiments to measure time reference PPG signals from organs which are not subject to pressure conditions **(71r** and **81r** respectively *i.e.,* cuff-free organ). For example, in possible embodiments the reference **PPG** probe **70r** may include an infrared light source (LS_{IR0}) **74r** and a light detector (Det₀) **77r,** configured to measure the time reference PPG signal from a finger **71r** in the contralateral hand of the examined subject in a transmission-mode configuration. These time reference PPG signals may be utilized to improve reliability of the detection of the reappearance of the PPG pulses in the PPG signals measured in the examined tissues **71m** and **81m.** More particularly, the time-reference PPG signals may assist in differentiating between the faint PPG pulses reappearing once the pressure applied over the organ decreases below the SBP, and changes in the light transmission curve, which are due to background noise. Alternatively, or additionally, pressure pulses in the pressure cuff and/or the ECG R-wave may also be used as a time-reference to assist in differentiating between weak reappearing PPG pulses and changes which are due to background noise.

In these examples the PPG probes, **70** and **80,** are designed to measure blood pressure using a single detector Det₁ **(77** and **87** respectively) and two light sources LS_{V} and LS_{IRIS} (*e.g.,* light emitting diodes - LEDs). The LS_{V} light sources, **72** and **82,** are configured to illuminate in the visible spectral range (*e.g.,* 450 to 506 nm) and are used for recording PPG signals responsive to passage of light in the visible spectral range through superficial regions of the examined tissues, **71m** and **81m** respectively. The LS_{IRIS} light sources, **74** and **84,** and the LS_{IR0} light sources, **74r** and **84r,** are respectively configured to illuminate light in the infrared isosbestic point (*e.g.,* about 800 nanometers), and are used for recording PPG signals responsive to passage of light in the infrared spectral range through relatively deep regions of the examined tissues, **71m** and **81m** respectively.

In both examples, the PPG probes **70** and **80** are designed to measure light emitted from the visible light sources, LS_{V} **72** and **82** respectively and reflected *(i.e.,* reflection-mode configuration) from the examined tissues. Due to the high absorption of the visible light in blood (see **Fig. 5****)** the light detectors **77** and **87** of the PPG probes **70** and **80** respectively, are located adjacent to the visible light sources, LS_{V} **72** and **82** respectively (*e.g.,* at a distance of 2 to 7 mm) therefrom.

With reference to **Fig. 7****,** a control unit **76** may be used to operate the PPG probe **70** and a pressure applying device **78.** The pressure applying device **78** comprises a pressure cuff **88** placed over the arm (not shown) of the examined subject and a pressure unit **63** comprising a pressure pump configured to operate the cuff **88** to apply pressure over the arteries upstream to the finger **71m** and a pressure measuring transducer to measure the pressure in the cuff. In this example the control unit **76** is configured to operate the LS_{IRIS} light source **74** in a transmission-mode by generating respective control signals **76i,** the LS_{V} **72** light source in a refraction-mode by generating respective control signals **76v,** and receive respective measured light intensity transmission/reflection signals **76m** (also referred to herein as optical data) from the detector Det₁ **77.** The control unit **76** may be configured to activate the pressure unit **63** to apply varying pressure conditions (*e.g.,* as exemplified by curve **20** in **Fig. 2****)** by the pressure cuff **88** over the arm of the examined subject by producing respective control signals **76e,** and to receive measured pressure data **76p** from the pressure measuring transducer in the pressure unit **63** indicative of the pressure applied over the arm.

In possible embodiments the control unit **76** is further configured to simultaneously operate the reference PPG probe **70r.** More particularly, the control unit **76** may be configured to operate the LS_{IR0} **74r** light source simultaneously during the same time periods in which the varying pressure conditions are applied by the pressure device **78** by producing corresponding control signals **76f to** illuminate the finger **71r** in the contralateral hand (or another pressure free limb or organ) of the subject, and receive corresponding measured light transmission signals **76r** (also referred to herein as reference optical data) from the detector Det₀ **77r.**

Similarly, the control unit **86** shown in **Fig. 8** may be configured to operate the PPG probe **80** and the pressure device **78,** comprising the pressure cuff **88** placed over the neonate leg **81g** and configured to apply pressure over the arteries upstream to the foot **81m.** In this example the control unit **86** is configured to operate the LS_{IRIS} light source **84** and the LS_{V} **82** light source in a refraction-mode by generating respective control signals **86i** and **86v,** and receive respective measured light intensity reflection signals **86m** from the detector Det₁ **87.** The control unit **86** may be configured to activate the pressure pump in the pressure device **78** to apply varying pressure conditions (*e.g.,* as exemplified in **Fig. 2****)** by the pressure cuff **88** over the leg **81g** of the neonate by producing respective control signals **86e,** and to receive measured pressure data **86p** from the pressure measuring transducer in the pressure device **78** indicative of the pressure applied over the leg **81g.**

In possible embodiments the control unit **86** is further configured to simultaneously operate the reference PPG probe **80r.** More particularly, the control unit **86** may be configured to operate the LS_{IR0} **84r** light source during the same time periods in which the varying pressure conditions are applied by the pressure device **78** by producing corresponding control signals **86f** to illuminate the contralateral foot **81r** (or another pressure free limb or organ of the neonate) and receiving corresponding measured light transmission signals **86r** from the detector Det₀ **87r.**

In some possible embodiments the PPG probes **(70** and **80)** may comprise an additional infrared light source configured to illuminate the examined tissue with light in the 850-900 nm range (above the infrared isosbestic point). For example, in the PPG probes **70** designed for adult subjects the additional infrared light source **79** may be arranged in a transmission-mode. The control unit **76** may be further configured, in some embodiments, to operate the additional infrared light source **79** in the transmission mode by producing respective control signals **76q,** and receive corresponding measured light transmission intensity signals over line **76m** from the light detector **77.** In the neonate PPG probe **80** design the additional infrared light source **89** may be arranged, in some embodiments, in a reflection-mode *e.g.,* at a distance of 10 to 20 millimeters from the light detector **87.** Accordingly, the control unit **86** may be configured to operate the additional infrared light source **89** in the reflection mode by producing respective control signals **86q,** and receive corresponding measured light reflection intensity signals over line **86m** from the light detector 77.

The control unit **(76** and **86)** may be configured to use the additional infrared light source **(79** and **89)** to detect the PPG pulses when the pressure applied by the pressure device decreases below the SBP value, and to use the near isosbestic infrared light source (LS_{IRIS}, **74** and **84)** for the detection of the declination of the DC light transmission/refraction curve. As in previously described embodiments, the control unit may be configured to use the light source in the visible range (LS_{V}, **72** and **82),** together with the same detector **(77** and **87)** for detecting the reappearance of the PPG pulses when the pressure applied by the pressure device decreases below the SBP value.

Accordingly, in some embodiments, the control unit may be configured to operate the various light sources in the PPG probes **70** and **80 (72, 74** and **82, 84** respectively) in a time-sharing (multiplexing) mode or continuous mode, and correspondingly generate from the PPG signals obtained two or single AC curves of PPG pulses, reflecting the light transmission/reflectance in the 450-500 nm (blue) range and in the infrared range, and a raw light transmission curve for the near (isosbestic) 800 nm (IR) light.

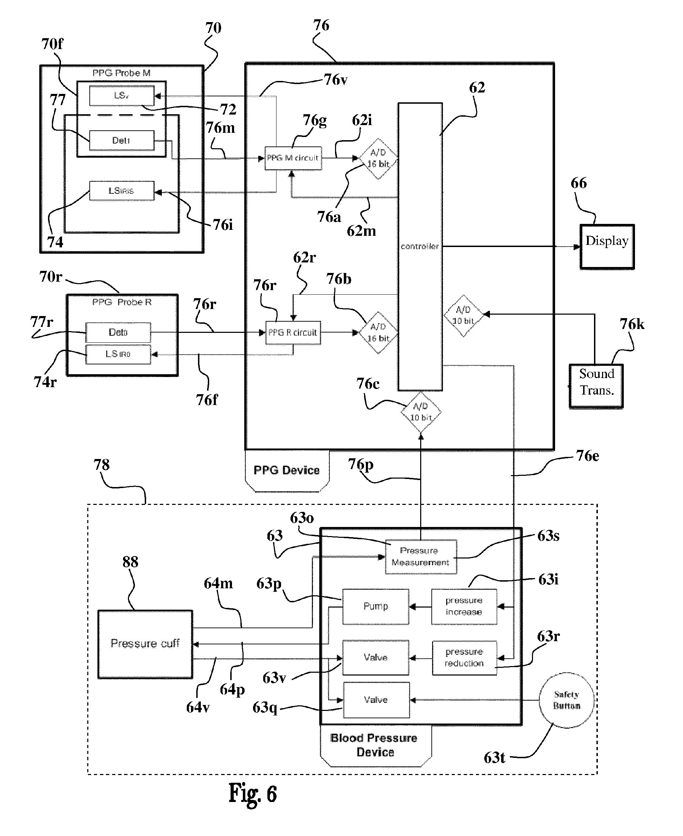

**Fig. 6** is a block diagram illustrating a system using the control unit **76** for determining systolic blood pressure of a subject using the PPG probe **70** for measuring PPG signals from the examined tissue and the pressure device **78** for applying descending pressure conditions to an organ upstream to the examined tissue. The control unit **76** in this example comprises a controller **62** and a PPG signal measurement unit **76g** configured to operate the PPG sensor **70** responsive to control signals **62m** received from the controller **62.**

For example, the PPG signal measurement unit **76g** may be configured to issue the control signals **76v** and **76i,** to activate the LS_{V} **72** and LS_{IRIS} **74,** light sources respectively, responsive to one or more control signals **62m** from the controller **62,** and optionally amplify and/or filter (if so needed), respective light transmission/refraction intensity signals **76m** received from the detector Det₁ 77 responsive to light from the light sources. The PPG signal measurement unit **76g** is further configured to transfer the received light intensity signals **76m** to a sampling unit **76a** (A/D) configured to digitize the light intensity signals and provide the resulting digital data to the controller **62.**

The controller **62** in this example is further configured to issue pressure control signals **76e** for activating the pressure unit **63** of the pressure device **78,** and a sampler unit **76c** configured to digitize pressure signals **76p** indicative of the pressure applied by the pressure cuff **88** over the organ and provide the resulting digitized data to the controller **62.** More particularly, in some embodiments the pressure device **78** may comprise a pressure cuff **88** coupled to pressure unit **63** via a pressure injection line **64p** configured to communicate pressure between the pump **63p** and the cuff **88** in the pressure unit **63** to inflate the cuff **88,** and a pressure discharge line **64v** configured to discharge pressure from the cuff **88** through a controlled valve **63v** and a safety valve **63q** provided in the pressure unit **63** to discharge pressure from the cuff **88.**

The pressure unit **63** may further comprise a pressure increasing unit **63i** configured to operate the pump **63p** responsive to control signals **76e** from the controller **62** instructing the pressure unit **63** to increase the pressure in the cuff **88,** and a pressure reducing unit **63r** configured to operate the controlled valve **63v** to discharge pressure from the cuff **88** responsive to control signals **76e** from the controller **62** instructing the pressure unit **63** to reduce the pressure in the cuff. The pressure cuff **88** is connected by a tube **64m** to a pressure measurement unit **63s** provided in the pressure unit **78** and comprising a pressure sensor **63o.** The pressure measurement unit **63s** is configured to generate pressure measurement signals **76p,** optionally amplify and/or filter (if so needed) the pressure measurement signals **76p,** and provide the pressure signals **76p** to the control unit **76** over a pressure measurement line. The safety valve **63q** in some embodiments may be a normally-closed pressure valve configured to discharge cuff pressure by manual activation of a safety button **63t** by a user, to thereby permit the user to change the state of the safety valve **63q** into an open state, if so needed.

The control unit **76** may further comprise in some embodiments a display device **66** on which processed data from the controller **62,** such as systolic blood pressure and heart rate, are displayed. Additionally or alternatively, the control unit **76** may comprise a USB controller configured to exchange data with an external computer system.

In some embodiments the control unit **76** may further include a PPG reference signal measurement unit **76r,** configured to operate the additional PPG probe **70r** coupled to a pressure-free organ responsive to control signals **62r** received from the controller **62.** More particularly, the PPG reference signal measurement unit **76r** may be configured to issue the control signal **76f** to activate the LS_{IR0} light source of the additional PPG probe **70r** responsive to control signal **62r** from the controller **62,** receive, and optionally amplify and/or filter (if so needed), light transmission intensity signals **76r** from the detector Det₀ **77r** and transfer the same to a sampling unit **76b** configured to digitize the received light intensity signal and provide the resulting digital data to the controller **62.**

The controller **62** may comprise a processor and memory devices for storing programs and other data for operating the units **76, 70, 70r,** and **78** of the system. For example, the controller **62** may be configured to operate the PPG probe **70** (and optionally also the reference PPG probe **70r)** and the pressure device **78,** process the optical and pressure data responsively received, and determine the systolic pressure of the examined subject using one or more of the methods described hereinabove or hereinbelow. The controller **62** may be further configured to determine the presence and lengths of the time delays ***Δt*** (see **Figs. 3** and **4****)** between pulses identified distal to the cuff in the signals received from the PPG probe **70** and the PPG pulses identified in the cuff-free organ in the signals received from the reference PPG probe **70r.** The determined time delays ***Δt*** may be used by the controller **62** to improve the process of differentiating between changes associated with the reappearance of the PPG pulses and the changes which are due to interfering noise.

In some embodiments the controller **62** may comprise an input to receive signals from sound transducer **76k** (*e.g.,* piezoelectric transducer) that is located under the cuff **88** and configured and operable to detect the Korotkoff sounds, and produce responsive audible data. The controller **62** may be configured to process the audible (Korotkoff sounds) data together with the optical and pressure data responsively received, and determine the systolic pressure of the examined subject using one or more of the methods described hereinabove or hereinbelow.

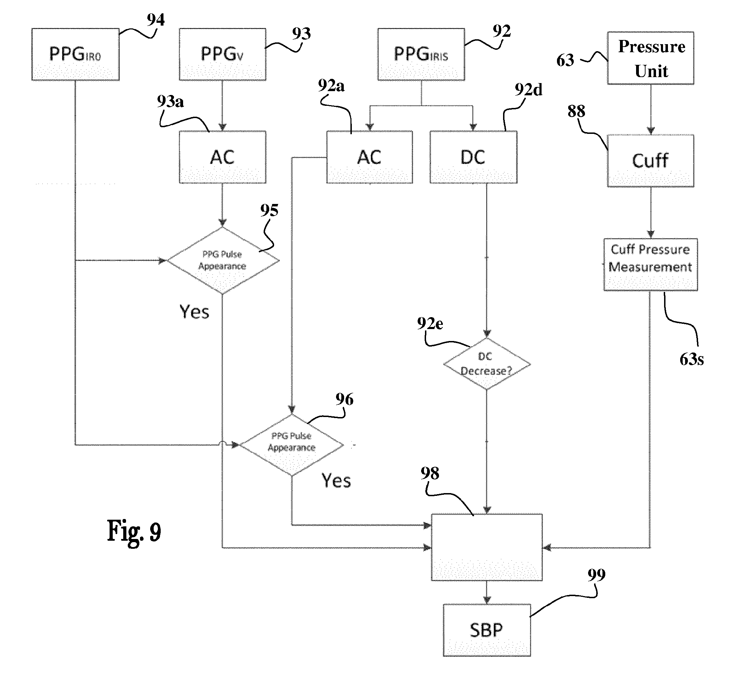

**Fig. 9** is a block diagram exemplifying various techniques for measuring systolic blood pressure according to some embodiments of the present application. According to one possible embodiment a single light source is used in the PPG probe to illuminate infrared isosbestic light on the examined tissue while gradually releasing the pressure applied by cuff **88** upstream to the examined tissue, starting from a pressure level greater than the systolic blood pressure. The optical data obtained via the light detector of the PPG probe responsive to light emitted by the infrared isosbestic light source is used to generate a PPG_{IRIS} curve **92.** The PPG_{IRIS} curve **92** is used in this example to determine a pulsatile component **92a** analyzed in a PPG pulse identifying unit **96** configured to identify reappearance of the PPG pulses and issue a responsive indication, and a baseline (DC) component **92d** analyzed in a declination identifying unit **92e** configured to identify commencement of declination of the baseline component **92d** and issue a responsive indication. The indication issued by the pulse identifying unit **96** and the declination identifying unit **92e** are received by a decision unit **98** configured to simultaneously receive pressure data from the pressure measurement unit **63s** and determine systolic blood pressure **99** as the cuff pressure at which at least one indication was received from the identifying units **96** and **92e.**

In some embodiments the PPG probe includes an additional light source configured to illuminate light in the visible range (*e.g.,* 450-500 nm wavelength range) on the examined tissue. The optical data obtained via the light detector of the PPG probe responsive to light illuminated by the visible light source is used to further generate a corresponding PPGᵥ curve **93.** The PPGᵥ curve **93** is used in this example to determine a pulsatile component **93a** analyzed in a PPG pulse identifying unit **95** configured to identify reappearance of the PPG pulses and issue a responsive indication. The indication issued by the pulse identifying unit **95** is received by the decision unit **98** which determines the systolic blood pressure value **99** as the cuff pressure received from the pressure measurement unit **63s** at which at least one indication was received from the identifying units **96, 92e** and **95.**

In some embodiments an additional PPG probe may be used to measure pressure free reference PPG signals from an organ (*e.g.,* in a contralateral limb) of the examined subject. The optical data obtained by the additional PPG probe may be used to generate a reference pressure free PPG_{IR0} curve **94.** In this example the pulse identifying units **95** and **96** are further configured to receive the pulses in the reference pressure free PPG_{IR0} curve **94** and use them as a guiding reference to differentiate between changes identified in the pulsatile components which are due to the reappearance of the PPG pulses and the changes which are due to interfering noise induced in the pulsatile components. In this way the accuracy of the systolic blood pressure value **99** determined by the decision unit **98** is further improved.

The pulse identifying units **95** and **96** may be further configured to determine the presence and the lengths of the time delays ***Δt*** (see **Figs. 3** and **4****)** between the PPG pulses identified in the pulsatile components **92a** and **93a** *(i.e.,* distal to the cuff) and the PPG pulses in the reference pressure free PPG_{IR0} curve **94** *(i.e.,* in the cuff-free organ). Detection of the presence and the lengths of the time delays ***Δt*** may be used in some embodiment to improve accuracy of the differentiating functionality (between changes associated with the reappearance of the PPG pulses and the changes which are due to interfering noise) of the pulse identifying units **95** and **96.** For example, in some embodiments the presence of time delays ***Δt*** of suitable lengths *(e.g.,* in the range of 100-200 milliseconds) indicates the appearance of PPG pulses in the tissue distal to the cuff.

In some embodiments the pressure device is configured and operable to apply ascending pressure conditions over the examined organ, and generate pressure data indicative of the pressure applied over said organ. The control unit may be accordingly configured to detect in the pulsatile component, baseline component, and/or measured Korotkoff sounds, the vanishing of the blood pulses once the pressure applied over the examined organ becomes greater than the systolic blood pressure, identify pressures values from the pressure data at which the vanishing of the blood pulses has been detected in the pulsatile component, baseline component, and/or measured Korotkoff sounds, and determine the systolic blood pressure accordingly. For example, the control unit may be configured to determine the systolic blood pressure as the maximal identified pressure at which the blood pulses can be detected in at least one of the pulsatile component, baseline component, and/or measured Korotkoff sounds.

The PPG-based measurement techniques of the present application are of particular importance for SBP measurement in the following scenarios:
- In populations who cannot always demonstrate Korotkoff sounds such as infants, and, in particular, neonates. In neonates blood pressure measurement is generally done through oscillometry, which is not accurate, and the PPG-based technique can replace it.
- The problem of weak or absent Korotkoff sounds also appears in patients with very low blood pressure as often occurs in cardiac intensive care units.
- In noisy environments such as in an ambulance or helicopter, where Korotkoff sounds cannot be properly heard.
- In the lower limbs in which Korotkoff sounds are generally absent. The ankle/brachial pressure ratio is clinically important for the assessment of stenosis in the lower limbs.
- As a supplemental method to sphygmomanometry. Simultaneous measurements of sphygmomanometry and the PPG-based technique can provide more accurate assessment of SBP than each method taken alone.
- As an automatic accurate method for systolic blood pressure measurement. The available commercial devices for automatic blood pressure measurement are based on oscillometry, which is not accurate and in general is not based on sphygmomanometry, due to artifacts. The automatic PPG-based techniques of the present application can provide accurate assessment of SBP.

The above examples and description have of course been provided only for the purpose of illustration, and are not intended to limit the invention in any way. As will be appreciated by the skilled person, the invention can be carried out in a great variety of ways, employing more than one technique from those described above, all without exceeding the scope of the invention.

## Claims

1. A system for determining systolic blood pressure of a subject, comprising:
a pressure device configured and operable to apply monotonic changing pressure conditions over a region of an organ of the subject, and generate pressure data indicative of the pressure applied over said organ;
an optical probe comprising at least one light source configured and operable to illuminate a tissue in said organ and distal to the said region of said organ with light, and at least one light detector configured and operable to detect light response of said tissue to the illuminated light and generate optical data indicative thereof;
a control unit configured and operable to operate said pressure device to apply said monotonic changing pressure conditions over the said region of said organ over a range of pressures including the systolic blood pressure and simultaneously operate the optical probe to illuminate said tissue and generate the responsive optical data, and to analyze the optical data and determine at least one pulsatile component and at least one baseline component therefrom, identify in said pulsatile and baseline components appearance of changes related to heart-induced change in blood volume in the said region of said organ in each one of said pulsatile and baseline components, determine from the pressure data a highest pressure value for each of said pulsatile and baseline components at which the identified changes are related to the blood volume changes in the organ, and determine the systolic blood pressure as the maximum value from the determined highest pressure values.

2. A system according to Claim 1 wherein the pressure conditions applied over the region of the organ are one of: descending pressure conditions starting from a pressure greater than the systolic blood pressure; or ascending pressure conditions starting from a pressure smaller than the systolic blood pressure.

3. A system according to any one of the preceding Claims wherein the optical probe comprises a light source configured and operable to illuminate light in a wavelength near an isosbestic wavelength point in the infrared region.

4. A system according to Claim 3 wherein the optical probe further comprises a light source configured and operable to illuminate light in the visible wavelength range of 400 to 600 nanometers.

5. A system according to Claim 4 wherein the visible wavelength light source is configured and operable to illuminate light in the wavelength range of 450 to 506 nm.

6. A system according to any one of Claims 5 wherein the control unit is configured and operable to determine only the at least one pulsatile component responsive to the illuminated visible light.

7. A system according to any one of the preceding claims comprising an additional optical probe comprising at least one light source configured and operable to illuminate a tissue in another organ, or another organ site, of the subject, not being affected by the pressure applied by the pressure device, and at least one light detector configured and operable to detect light response of said tissue to the illuminated light and generate reference optical data indicative thereof, wherein the control unit is configured and operable to determine a pulsatile component from the reference optical data and utilize it to discriminate between pulsatile changes which are responsive to the heart-induced change in blood volume in the organ and changes that are due to noise.

8. A system according to any one of Claims 1 to 7 wherein the optical probe comprises at least one light source configured and operable to illuminate light in a wavelength above the infrared isosbestic wavelength point.

9. A system according to Claim 8 wherein the control unit is configured and operable to determine the at least one pulsatile component responsive to the above isosbestic wavelength point light illumination and the at least one baseline component responsive to the near isosbestic wavelength point illuminated light.

10. A system according to any one of the preceding Claims comprising a sound transducer configured and operable to sense audible signals in the organ region while the changing pressure conditions are being applied and generate audible data indicative thereof, wherein the control unit is configured to process the audible data and detect Korotkoff sounds therein, and wherein the control unit is configured to determine from the pressure data the highest pressure at which said Korotkoff sounds started to appear and determine the systolic blood pressure as the maximum value from the highest pressure values determined for the pulsatile component, the baseline component, and the Korotkoff sounds.

11. A system for determining systolic blood pressure of a subject, comprising:
a pressure device configured and operable to apply descending pressure conditions over a region of an organ of the subject, and generating pressure data indicative of the pressure applied over said organ;
an optical probe comprising at least two light sources configured and operable to illuminate a tissue in said organ and distal to the pressure device with light in the visible range and in a wavelength near an infrared isosbestic wavelength point, and at least one light detector configured and operable to detect light response of said tissue to the illuminated light and generate optical data indicative thereof;
a sound transducer configured and operable to sense audible signals in the organ region while the descending pressure conditions are being applied and generate audible data indicative thereof;
a control unit configured and operable to operate said pressure device to apply descending pressure conditions over the said region of said organ starting from a pressure greater than the systolic blood pressure and simultaneously operate the optical probe to illuminate the tissue and generate the responsive optical data, to analyze the optical data and determine at least one pulsatile component responsive to the illuminated visible light and at least one baseline component responsive to the illuminated near isosbestic wavelength point light, identify appearance of changes in each one of said pulsatile and baseline components, determine from the pressure data a highest pressure value at which the appearance of the Korotkoff sounds occurred, determine from the pressure data a highest pressure value for each of said pulsatile and baseline components at which the identified changes are responsive to increase of blood volume in the organ, and determine the systolic blood pressure as the maximum value from the determined highest pressure values.

12. A system according to Claim 11 wherein the visible wavelength light source is configured and operable to illuminate light in the wavelength range of 450-506 nm.

13. A method of determining systolic blood pressure in a subject, comprising:
applying monotonic changing pressure conditions, over a range of pressures including the systolic blood pressure, over a region of an organ of said subject and simultaneously illuminating a tissue in the pressurized organ with light;
measuring optical data indicative of passage of the light through the tissue and pressure data indicative of the pressure being applied over the said region of said organ;
determining at least one pulsatile component and at least one baseline component from the measured optical data;
identifying in each of the components changes indicative that the pressure applied over the organ is smaller than a systolic blood pressure of the subject; and
determining the systolic blood pressure of the subject as a maximal applied pressure at which at least one of the following started to appear: the changes in the pulsatile component; and the changes in the baseline component.

14. The method according to claim 13 further comprising:
illuminating another tissue of the subject with light, said tissue not being under the applied pressure conditions;
measuring optical data indicative of passage of the light through said another tissue;
determining an additional pulsatile component from the optical data measured from said another tissue and using it as a reference to discriminate the changes indicative that the pressure applied over the organ is smaller than the systolic blood pressure of the subject from noise in the pulsatile component measured for the tissue in the organ to which the changing pressure conditions are being applied.

15. The method of claim 13 or 14, comprising detecting Korotkoff sounds in the organ to which the changing pressure conditions are being applied, wherein the determining includes determining the systolic blood pressure of the subject as a maximal applied pressure at which at least one of the following started to appear: said Korotkoff sounds; the changes in the pulsatile component, and the changes in the baseline component.
